# EUROPEAN PATENT APPLICATION

(11) **EP 2 974 703 A1**
(43) Date of publication of application: **20.01.2016**
(21) Application number: 14177441.4
(22) Date of filing: 17.07.2014
(51) Int. Cl.: A61F 13/64, A61F 13/56

(54) **Diaper**

(71) Applicant: Cheng, Yuan-Long, Tainan City (TW)
(72) Inventor: Cheng, Yuan-Long, Tainan City (TW)
(74) Representative: Viering, Jentschura & Partner mbB Patent- und Rechtsanwälte

(57) **Abstract**

A diaper has a diaper body (10), two straps (20) and at least one strap hook-and-loop fastener (21). The diaper body (10) has an absorbent portion (11), a front covering portion (13) connected with the absorbent portion (11), at least one body hook-and-loop fastener (14) mounted on the front covering portion (13), and a rear covering portion (15) connected with the absorbent portion (11). The straps (20) are elongated and are respectively connected with two sides of the rear covering portion (15). The body hook-and-loop fastener (14) is selectively attached to the straps (20). The strap hook-and-loop fastener (21) is mounted on one of the straps (20), and the strap hook-and-loop fastener (21) is selectively attached to the other strap (20). Therefore, the diaper can be suitable for different users of different waist sizes.

## Description

### 1. Field of the Invention

The present invention relates to a diaper, and more particularly to a diaper that has an adjustable fixing structure.

### 2. Description of Related Art

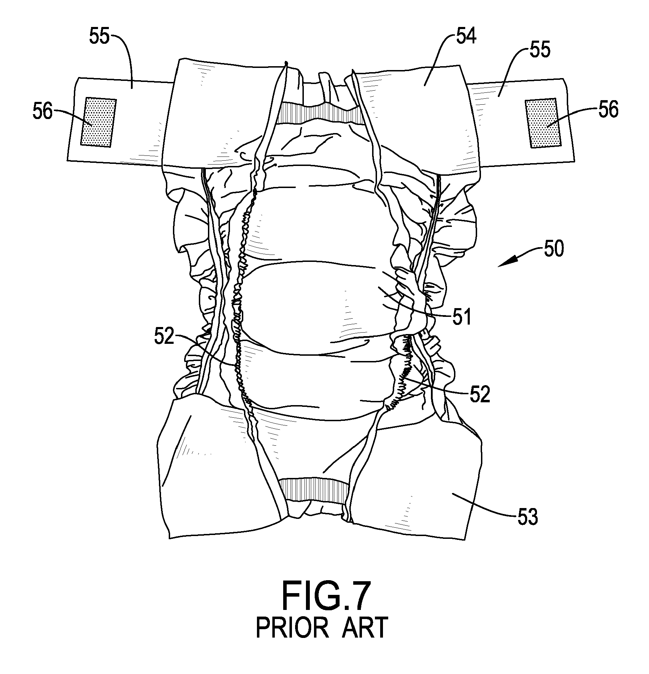

With reference to Fig. 7, a conventional diaper 50 has an absorbent portion 51, two leakproof guards 52, a front covering portion 53, a rear covering portion 54, two fixing straps 55 and two hook-and-loop fasteners 56. The leakproof guards 52 are respectively mounted on two opposite sides of the absorbent portion 51. The front covering portion 53 is connected with a front end of the absorbent portion 51. The rear covering portion 54 is connected with a rear end of the absorbent portion 51. The fixing straps 55 are respectively connected with two sides of the rear covering portion 54. The hook-and-loop fasteners 56 are respectively mounted on the fixing straps 55.

In use, the absorbent portion 51 is placed under a crotch of a user. The front covering portion 53 is placed in front of an abdomen of the user, and the rear covering portion 54 is placed in rear of the buttocks of the user. Then, the fixing straps 55 are pulled frontward to approach and cover part of the front covering portion 53, such that the hook-and-loop fasteners 56 mounted on the straps 55 can be attached to the front covering portion 53. Therefore, the diaper is worn on the user.

However, the fixing straps 55 cannot be wrapped around the waist of the user because the fixing straps 55 are not long enough to contact and be attached to each other. Therefore, the diaper is unsuitable for different users with different waist sizes. Furthermore, when the user is involved in intensively active movements, the diaper may be detached from the user. The described problems have to be resolved.

The main objective of the present invention is to provide a diaper to resolve the aforementioned problems.

The diaper of the present invention has a diaper body, two straps and at least one strap hook-and-loop fastener.

The diaper body has an inner surface, an absorbent portion formed on the inner surface, a front covering portion connected with an end of the absorbent portion, at least one body hook-and-loop fastener mounted on the front covering portion and located at the inner surface of the diaper body, and a rear covering portion connected with another end of the absorbent portion at a position opposite to the front covering portion, and the rear covering portion located at the inner side of the diaper body.

The two straps are elongated and are respectively connected with two sides of the rear covering portion. Each strap has an inner side and an outer side. The inner side of each strap is connected with the inner surface of the diaper body. The outer side of each strap is opposite to the inner side. The at least one body hook-and-loop fastener is selectively attached to the outer side of one of the straps.

The at least one strap hook-and-loop fastener is mounted on one of the inner side and the outer side of one of the straps, and the at least one strap hook-and-loop fastener is selectively attached to the other strap.

Other objectives, advantages and novel features of the present invention will become more apparent from the following detailed description when taken in conjunction with the accompanying drawings.

### IN THE DRAWINGS

Fig. 1 is a perspective view of a first preferred embodiment of a diaper in accordance with the present invention;
Fig. 2 is a top view of the diaper in Fig. 1, shown spread;
Figs. 3 to 5 are operational views of the diaper in Fig. 1;
Fig. 6 is a top view of a second preferred embodiment of a diaper in accordance with the present invention; and
Fig. 7 is a top view of a conventional diaper.

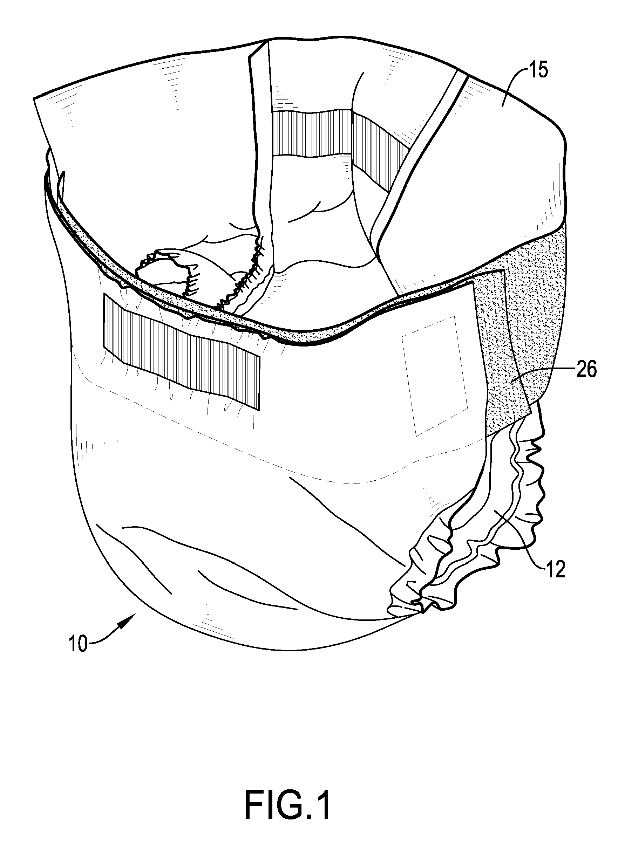

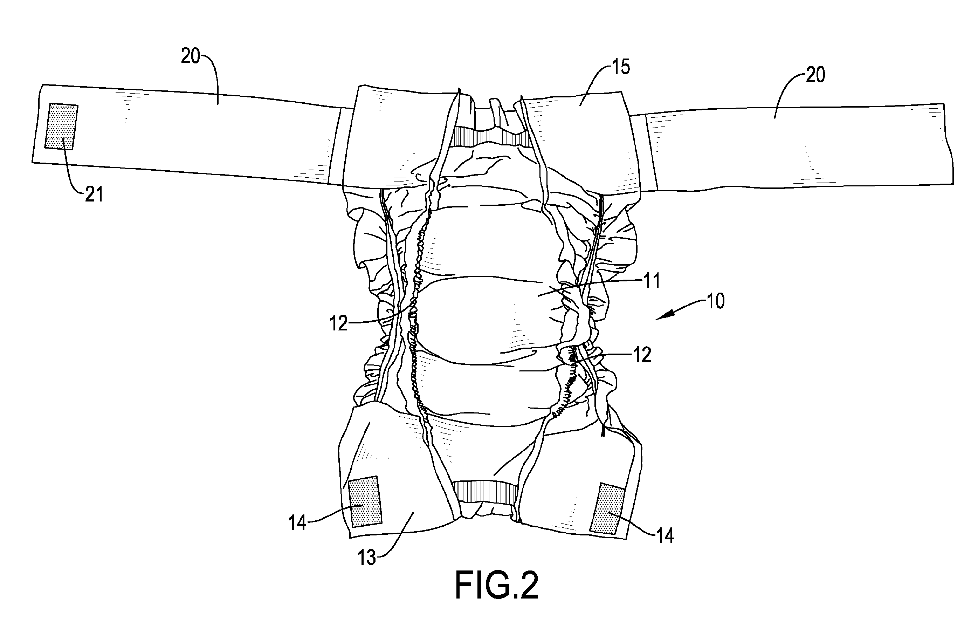

With reference to Figs. 1 and 2, a first preferred embodiment of a diaper in accordance with the present invention has a diaper body 10, two straps 20, and at least one strap hook-and-loop fastener 21.

The diaper body 10 has an absorbent portion 11, two leakproof guards 12, a front covering portion 13, two body hook-and-loop fasteners 14, and a rear covering portion 15. The absorbent portion 11 is formed on a middle and an inner surface of the diaper body 10 and is provided for absorbing excreta of a user. The leakproof guards 12 are respectively mounted on two opposite sides of the absorbent portion 11 and are also located at an inner surface of the diaper body 10. The leakproof guards 12 can keep the excreta from flowing out of the absorbent portion 11. The front covering portion 13 is connected with the absorbent portion 11 and is located at a front end of the diaper body 10. The body hook-and-loop fasteners 14 are mounted on the front covering portion 13 at an interval and are located at the inner surface of the diaper body 10. The rear covering portion 15 is connected with the absorbent portion 11, is located at a rear end and the inner surface of the diaper body 10.

The straps 20 are elongated and are respectively connected with two sides of the rear covering portion 15. Preferably, the straps 20 are made of nonwoven fabrics. Each strap 20 has an inner side and an outer side opposite the inner side, wherein the inner side of the strap 20 is connected with the inner surface of the diaper body 10.

The at least one strap hook-and-loop fastener 21 is mounted on a side of one of the straps 20. Preferably, the at least one strap hook-and-loop fastener 21 is mounted on the inner side of the corresponding strap 20. Alternatively, the at least one strap hook-and-loop fastener 21 may be mounted on the outer side of the corresponding strap 20. The at least one strap hook-and-loop fastener 21 is selectively attached to the other strap 20. The body hook-and-loop fasteners 14 are selectively attached respectively to the outer sides of the straps 20.

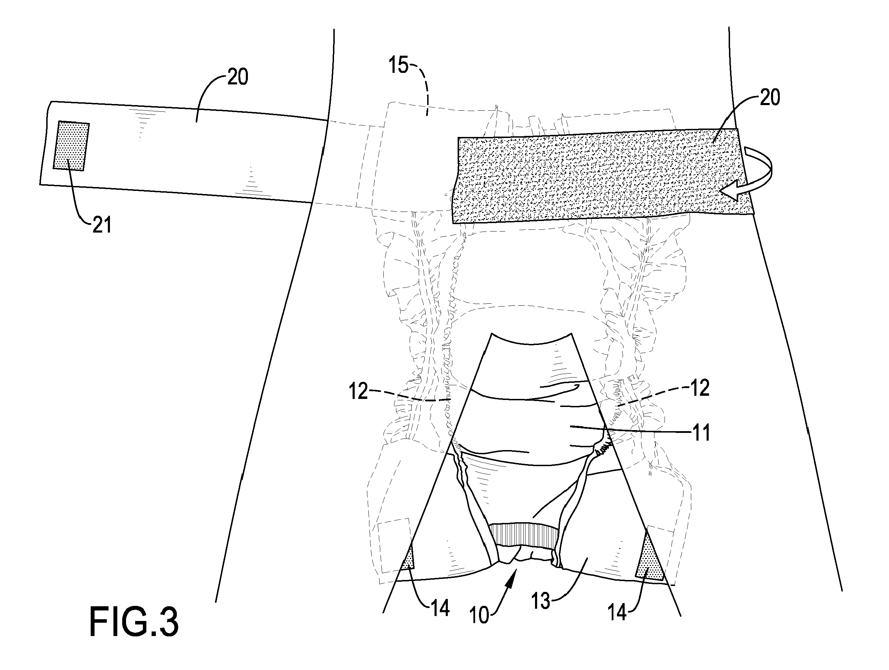

With reference to Figs. 3 to 4, in use, the straps 20 are wrapped around a waist of a user, and the rear covering portion 15 covers buttocks of the user. The straps 20 are attached to each other by the at least one strap hook-and-loop fastener 21. The strap hook-and-loop fastener 21 is attached to the outer side of the corresponding strap 20. Alternatively, when the strap hook-and-loop fastener 21 is mounted on the outer side of one of the straps 20, the strap hook-and-loop fastener 21 is attached to the inner side of the corresponding strap 20. The straps 20 are attached to each other to keep the diaper from being inadvertently detached from the waist of the user.

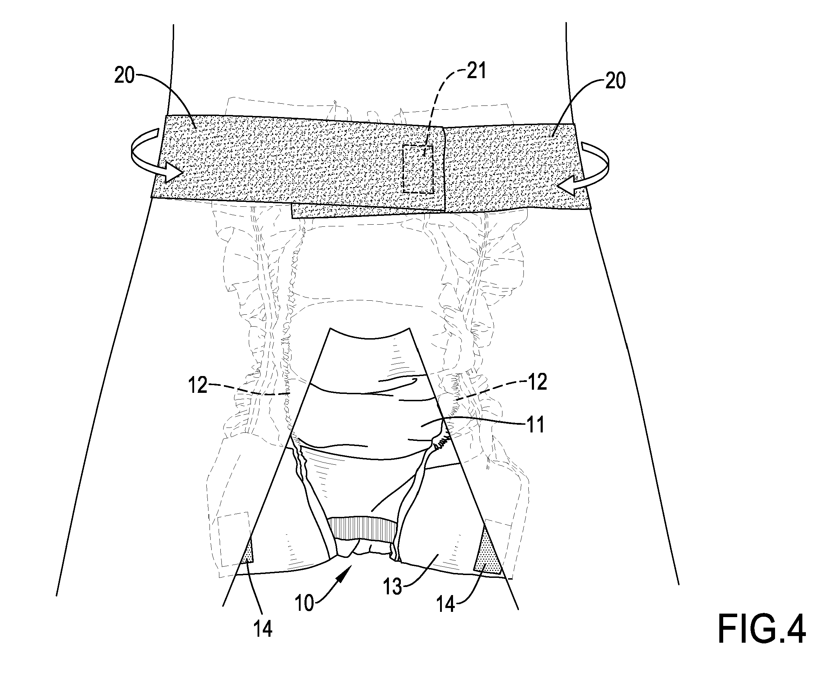

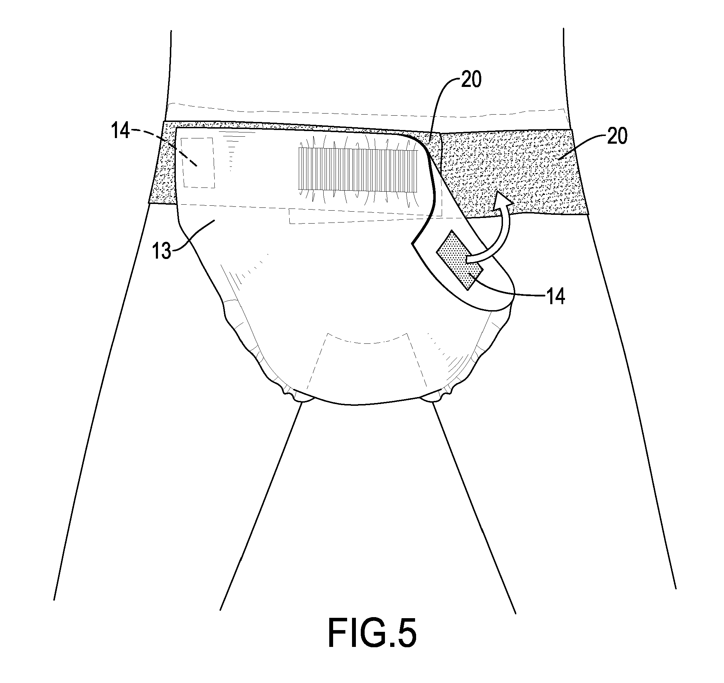

With reference to Figs. 4 and 5, after the straps 20 are attached to each other, the front covering portion 13 is crossed a crotch of the user and covers an abdomen of the user. The at least one strap hook-and-loop fastener 21 is attached to the outer sides of the straps 20 to keep the front covering portion 13 from detaching form the abdomen of the user. Therefore, the diaper is worn on the user.

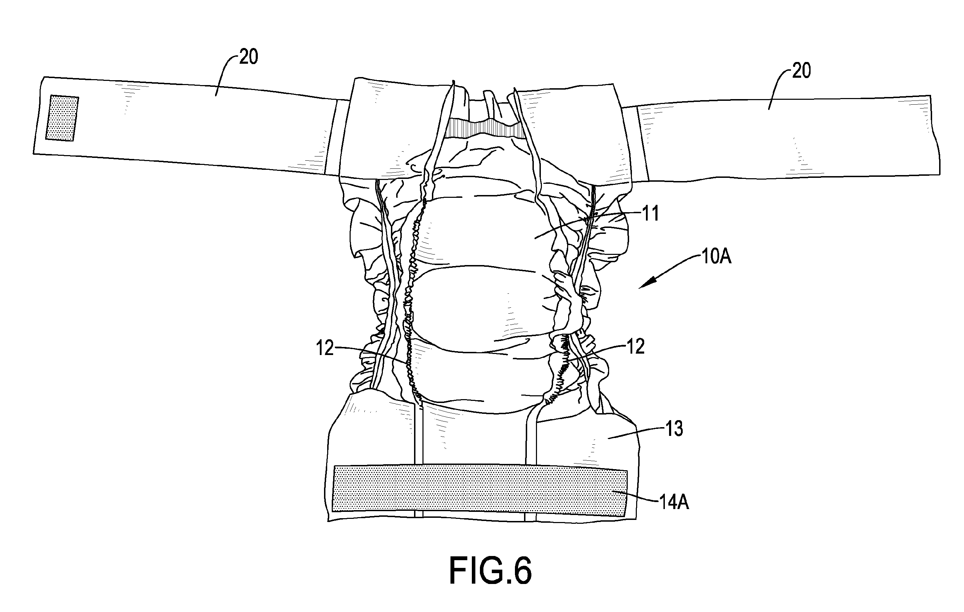

With reference to Fig. 6, a diaper body 10A of a second preferred embodiment of the diaper in accordance with the present invention only has one body hook-and-loop fastener 14A. The body hook-and-loop fastener 14A is elongated, is mounted on the front covering portion 13, and is located at the inner surface of the diaper body 10A. The method for use of the second preferred embodiment of the diaper is same with the front preferred embodiment of the diaper. The elongated body hook-and-loop fastener 14A can be attached to the straps 20 more firmly and the front covering portion 13 is less likely to be detached from the straps 20.

From the above description, it is noted that the present invention has the following advantages:
1.The straps 20 can be wrapped around the waist of the user, such that the diaper is suitable for different users with different waist sizes since the straps 20 can be wrapped in adjustment for different inner diameters.
2.The straps 20 are wrapped around the waist of the user and can further provide a firm fixing effect for the diaper, such that the diaper cannot be detached from the user easily. The straps 20 are made of nonwoven fabrics, such that the straps 20 can have a ventilation effect for the user.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A diaper, **characterized in that** the diaper has:
a diaper body (10) having
an inner surface;
an absorbent portion (11) formed on the inner surface;
a front covering portion (13) connected with an end of the absorbent portion (11);
at least one body hook-and-loop fastener (14) mounted on the front covering portion (13) and located at the inner surface of the diaper body (10); and
a rear covering portion (15) connected with another end of the absorbent portion (11) at a position opposite to the front covering portion (13), and located at the inner surface of the diaper body (10);
two straps (20) being elongated and respectively connected with two sides of the rear covering portion (15), wherein
each strap (20) has an inner side and an outer side;
the inner side of each strap (20) is connected with the inner surface of the diaper body (10);
the outer side of each strap (20) is opposite to the inner side of the strap (20); and
the at least one body hook-and-loop fastener (14) is selectively attached to the outer side of one of the straps (20); and
at least one strap hook-and-loop fastener (21) mounted on one of the inner side and the outer side of one of the straps (20), and the at least one strap hook-and-loop fastener (21) selectively attached to the other strap (20).

2. The diaper as claimed in claim 1, wherein the straps (20) are made of nonwoven fabrics.

3. The diaper as claimed in claim 2, wherein the at least one strap hook-and-loop fastener (21) is mounted on the inner side of one of the straps (20) and is selectively attached to the outer side of the other strap (20).

4. The diaper as claimed in any one of claims 1 to 3, wherein the diaper body (10) has two body hook-and-loop fasteners (14) mounted on the front covering portion (13) at an interval and selectively attached to the outer side of one of the straps (20).

5. The diaper as claimed in any one of claims 1 to 3, wherein the diaper body (10) has a body hook-and-loop fastener (14) mounted on the front covering portion (13) and selectively attached to the outer side of one of the straps (20).
